# EUROPEAN PATENT APPLICATION

(11) **EP 2 752 414 A1**
(43) Date of publication of application: **09.07.2014**
(21) Application number: 13150217.1
(22) Date of filing: 04.01.2013
(51) Int. Cl.: C07D 471/04, A61K 31/437, A61P 7/02

(54) **Crystalline form of apixaban**

(71) Applicant: Sandoz AG, 4056 Basel (CH)
(72) Inventor: The designation of the inventor has not yet been filed
(74) Representative: Kunic Tesovic, Barbara

(57) **Abstract**

The present invention relates to a crystalline polymorph Form A of Apixaban and the process for making it. Apixaban is useful as a direct inhibitor of activated factor (FXa) in the treatment of thromboembolic diseases.

## Description

### FIELD OF INDUSTRIAL APPLICABILITY

The present disclosure generally relates to a crystalline polymorphic Form A of Apixaban and to methods for obtaining such form. The present disclosure also generally relates to a pharmaceutical composition comprising Form A of Apixaban, as well to methods of using the Form A in the treatment of various thromboembolic diseases.

### BACKGROUND OF THE DISCLOSURE

Apixaban, 1-(4-methoxyphenyl)-7-oxo-6-(4-(2-oxopiperidin-1-yl)phenyl)-4,5,6,7-tetrahydro-1H-pyrazolo[3,4-c]pyridine-3-carboxamide), shown in Formula I is a pyrazolopyridine derivative that is known as a direct inhibitor of activated factor X (FXa), and is used in treatment of various thromboembolic diseases. As such, it has been disclosed in WO2003/026652.

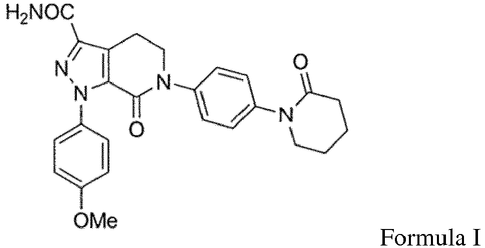

A patent applications US 2006/0069258 and WO2011/106478 discloses two polymorphic forms of apixaban, designated as Form N-1(neat) and Form H2-2 (hydrate). Another patent application US 2007/0203178 discloses crystalline solvated forms of apixaban, namely dimethyl formamide and formamide solvates designated as polymorph forms DMF-5 and FA-2, which are useful for preparing crystalline apixaban hydrate and neat forms.

For a pharmaceutical use there is a constant need for a preparation of active pharmaceutical ingredients having the amount of impurities and solvents as low as possible, as well as being physically stable during manufacturing of final dosage preparation and during storage up to its use for treatment in patients. Thus the solvate forms DMF-5 and FA-2 described in US2007/0203178 are due to the content of body unfriendly solvents not suitable for pharmaceutical use. The only form not characterized as solvate is apixaban Form N-1.

Another criterion which may be of exceptional importance under certain circumstances depending on the choice of formulation or the choice of manufacturing process is solubility of the active substance. For drugs which are to be taken orally it is, in general, very important that active pharmaceutical ingredients are sufficiently soluble and bioavailable. Apixaban is characterized by a low solubility in aqueous media. Thus, there is a need for preparation of apixaban polymorph form which has improved solubility. At the same time a physical stability of polymorph form is required.

### SUMMARY OF THE DISCLOSURE

The present invention provides a crystalline anhydrous polymorph form of Apixaban, characterized herein as apixaban Form A. The name used herein to characterize a specific form, e.g. "A", should not be considered limiting with respect to any other substance possessing similar or identical physical and chemical characteristics, but rather it should be understood that these designations are mere identifiers that should be interpreted according to the characterization information also presented herein.

In particular, the present invention relates to a novel crystalline form of the active compound apixaban Form A, an anhydrous form of apixaban, which is pharmaceutically acceptable in view of physical stability and equilibrium solubility.

Aspects of the invention are:
A crystalline Form A of apixaban.

The crystalline Form A of apixaban consisting essentially of Form A.

The crystalline Form A of apixaban, wherein said Form A is in substantially pure form.

The crystalline Form A of apixaban characterized by a x-ray powder diffraction pattern comprising five or more 2θ values selected from the group consisting of 3.9±0.2, 7.7±0.2, 8.5±0.2, 10.9±0.2, 11.6±0.2, 15.5±0.2, 17.9±0.2, 18.7 ±0.2, 20.6±0.2, 22.1±0.2 and 29.5±0.2.

The crystalline Form A of apixaban characterized by a x-ray powder diffraction pattern comprising 2θ values at 10.9±0.2, 17.9±0.2, 18.7 ±0.2, 20.6±0.2 and 22.1±0.2.

The crystalline Form A of apixaban characterized by a x-ray powder diffraction pattern comprising 2θ values at 3.9±0.2, 7.7±0.2, 8.5±0.2, 10.9±0.2, 11.6±0.2, 15.5±0.2, 17.9±0.2, 18.7 ±0.2, 20.6±0.2, 22.1±0.2, 29.5±0.2.

The crystalline Form A of apixaban characterized by a x-ray powder diffraction pattern comprising 2θ values at 3.9±0.2, 4.3±0.2, 6.2±0.2, 7.7±0.2, 8.5±0.2, 9.4±0.2, 10.9±0.2, 11.3±0.2, 11.6±0.2, 12.8±0.2, 13.8±0.2, 14.6±0.2, 15.5±0.2, 16.6±0.2, 17.2±0.2, 17.9±0.2, 18.7 ±0.2, 19.4±0.2, 20.6±0.2, 21.0±0.2, 22.1±0.2, 24.2±0.2, 27.0±0.2, 28.1±0.2, 29.5±0.2, 30.2±0.2 and 30.9±0.2.

The crystalline Form A of apixaban characterized by a x-ray powder diffraction pattern comprising 2θ values at 3.9±0.2, 4.3±0.2, 6.2±0.2, 7.7±0.2, 8.5±0.2, 9.4±0.2, 10.9±0.2, 11.3±0.2, 11.6±0.2, 12.8±0.2, 13.8±0.2, 14.6±0.2, 15.5±0.2, 16.6±0.2, 17.2±0.2, 17.9±0.2, 18.7 ±0.2, 19.4±0.2, 20.6±0.2, 21.0±0.2, 22.1±0.2, 24.2±0.2, 27.0±0.2, 28.1±0.2, 29.5±0.2, 30.2±0.2, 30.9±0.2, 33.1±0.2, 35.2±0.2, 36.8±0.2.

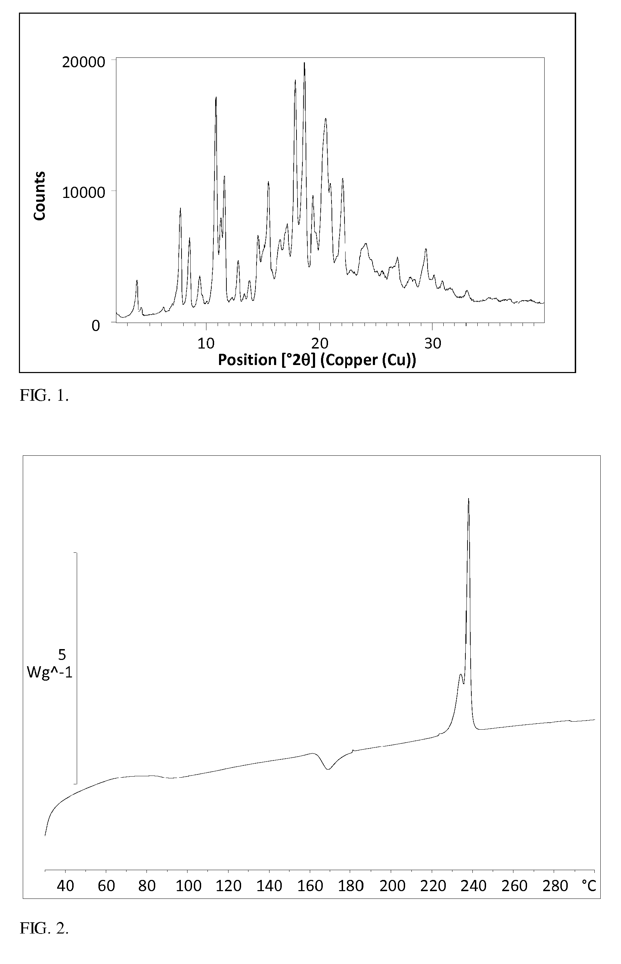

A crystalline Form A of apixaban having a X-ray diffraction spectrum substantially the same as the X-ray powder diffraction spectrum shown in FIG. 1.

A crystalline Form A of apixaban having a differential scanning calorimetry (DSC) thermogram substantially the same as that shown in shown in FIG. 2.

A pharmaceutical composition comprising crystalline Form A of apixaban and a pharmaceutically acceptable carrier or diluent.

The pharmaceutical composition comprising crystalline Form A of apixaban and a pharmaceutically acceptable carrier or diluent, wherein said Form A is in substantially pure form.

A pharmaceutical composition comprising the crystalline Form A of apixaban in combination with one or more therapeutic agents.

The pharmaceutical composition comprising the crystalline Form A of apixaban in combination with one or more therapeutic agents, wherein said Form A is in substantially pure form.

The pharmaceutical composition comprising at least 90 weight % of the crystalline Form A of apixaban, based on the weight of apixaban.

A method of treating a thromboembolic disease in a mammal comprising administering to the mammal a therapeutically-effective amount of a crystalline Form A of apixaban.

The method of treating a thromboembolic disease in a mammal comprising administering to the mammal a therapeutically-effective amount of a crystalline Form A of apixaban, wherein said Form A is in substantially pure form.

The method of treating a thromboembolic disease in a mammal comprising administering to the mammal a therapeutically-effective amount of a crystalline Form A of apixaban, wherein the mammal is a human.

A crystalline Form A of apixaban for use in treating a thromboembolic disease in a mammal.

A crystalline Form A of apixaban for use in treating a thromboembolic disease in a mammal comprising administering to the mammal a therapeutically-effective amount of the crystalline Form A of apixaban.

Use of a crystalline Form A of apixaban for the preparation of a pharmaceutical composition for use in the treatment of thromboembolic diseases.

A process of making a crystalline Form A of apixaban comprising:
a) heat apixaban Form H3 at elevated temperature; and
b) cooling the substance to a room temperature.

A process of making a crystalline Form A of apixaban comprising:
a) heat apixaban Form H3 at temperature from about 50°C to about 200°C, preferably from about 100°C to about 160°C, and more preferably at about 120°C in a time range from 10 min to 24 h, preferably from 30 min to 12 h, and more preferably from 1 h to 8 h; and
b) cooling the substance to a room temperature.

### BRIEF DESCRIPTION OF THE DRAWINGS

FIG. 1. illustrates the x-ray powder diffraction patterns of crystalline Form A of apixaban.
FIG. 2. illustrates the differential scanning calorimetry (DSC) of crystalline Form A of apixaban.

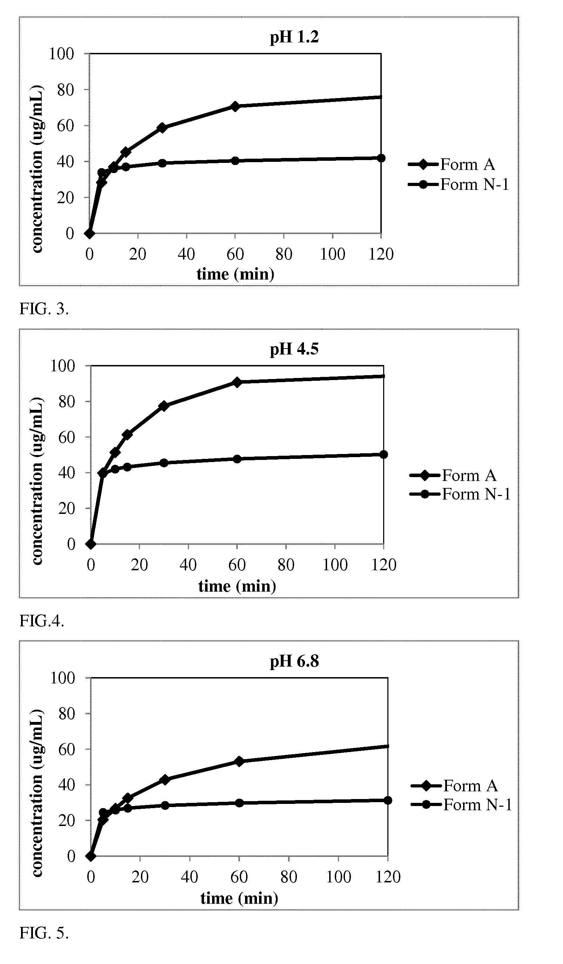

FIG. 3. illustrates a dynamic solubility of crystalline Form A and Form N-1 of apixaban in aqueous media of buffer of pH 1.2.
FIG. 4. illustrates a dynamic solubility of crystalline Form A and Form N-1 of apixaban in aqueous media of buffer of pH 4.5.
FIG. 5. illustrates a dynamic solubility of crystalline Form A and Form N-1 of apixaban in aqueous media of buffer of pH 6.8.
FIG. 6. illustrates the x-ray powder diffraction patterns of crystalline Form H3 of apixaban.

### DETAILED DESCRIPTION OF THE DISCLOSURE

The disclosure relates to crystalline Form A of apixaban, which are described and characterized herein.

### Definitions

As used herein "polymorph" refers to crystalline forms having the same chemical composition but different spatial arrangements of the molecules, atoms, and/or ions forming the crystal.

As used herein "solvate" refers to a crystalline form of a molecule that further comprises molecules of a solvent or solvents incorporated into the crystalline lattice structure. The solvent molecules in the solvate may be present in a regular arrangement and/or a non-ordered arrangement. The solvate may comprise either a stoichiometric or nonstoichiometric amount of the solvent molecules. For example, a solvate with a nonstoichiometric amount of solvent molecules may result from partial loss of solvent from the solvate.

As used herein "hydrate" refers to a crystalline form of a molecule that further comprises molecules of a water incorporated into the crystalline lattice structure. The water molecules in the hydrate may be present in a regular arrangement and/or a non-ordered arrangement. The hydrate may comprise either a stoichiometric or nonstoichiometric amount of the water molecules. For example, a hydrate with a nonstoichiometric amount of water molecules may result from partial loss of water from the hydrate.

As used herein "non-solvate" refers to a crystalline form of a molecule, *per se* that do not further comprise molecules of a solvent or solvents incorporated into the crystalline lattice structure, nor is it a salt formation.

As used herein "anhydrous" refers to a crystalline form of a molecule *per se* that do not further comprise molecules of water incorporated into the crystalline lattice structure, nor is it a salt formation.

The term "essentially the same" with reference to X-ray diffraction peak positions means that typical peak position and intensity variability are taken into account. For example, one skilled in the art will appreciate that the peak positions (2θ) will show some inter-apparatus variability, typically as much as 0.2°. Further, one skilled in the art will appreciate that relative peak intensities will show inter-apparatus variability as well as variability due to degree of crystallinity, preferred orientation, prepared sample surface, and other factors known to those skilled in the art, and should be taken as qualitative measure only.

As used herein, the term "substantially pure" with reference to a particular polymorphic form means that the polymorphic form includes less than 10%, preferably less than 5%, more preferably less than 3%, most preferably less than 1% by weight of any other physical forms of the compound.

As used herein, the term "equilibrium solubility" refers to the maximum solubility of apixaban in various aqueous media of buffers of pH 1.2, 4.5 and 6.8 at 37 °C after 24 hours. The shaking is carried out at 400 rpm.

The present invention provides the crystalline Form A of apixaban characterized by a x-ray powder diffraction pattern comprising five or more 2θ values selected from the group consisting of 3.9±0.2, 7.7±0.2, 8.5±0.2, 10.9±0.2, 11.6±0.2, 15.5±0.2, 17.9±0.2, 18.7 ±0.2, 20.6±0.2, 22.1±0.2 and 29.5±0.2.

Preferably, the crystalline Form A of apixaban is characterized by a x-ray powder diffraction pattern comprising 2θ values at 10.9±0.2, 17.9±0.2, 18.7 ±0.2, 20.6±0.2 and 22.1±0.2.

More preferably, the crystalline Form A of apixaban is characterized by a x-ray powder diffraction pattern comprising 2θ values at 3.9±0.2, 7.7±0.2, 8.5±0.2, 10.9±0.2, 11.6±0.2, 15.5±0.2, 17.9±0.2, 18.7 ±0.2, 20.6±0.2, 22.1±0.2, 29.5±0.2.

Even more preferably, the crystalline Form A of apixaban is characterized by a x-ray powder diffraction pattern comprising 2θ values at 3.9±0.2, 4.3±0.2, 6.2±0.2, 7.7±0.2, 8.5±0.2, 9.4±0.2, 10.9±0.2, 11.3±0.2, 11.6±0.2, 12.8±0.2, 13.8±0.2, 14.6±0.2, 15.5±0.2, 16.6±0.2, 17.2±0.2, 17.9±0.2, 18.7 ±0.2, 19.4±0.2, 20.6±0.2, 21.0±0.2, 22.1±0.2, 24.2±0.2, 27.0±0.2, 28.1±0.2, 29.5±0.2, 30.2±0.2 and 30.9±0.2.

Even more preferably, the crystalline Form A of apixaban is characterized by a x-ray powder diffraction pattern comprising 2θ values at 3.9±0.2, 4.3±0.2, 6.2±0.2, 7.7±0.2, 8.5±0.2, 9.4±0.2, 10.9±0.2, 11.3±0.2, 11.6±0.2, 12.8±0.2, 13.8±0.2, 14.6±0.2, 15.5±0.2, 16.6±0.2, 17.2±0.2, 17.9±0.2, 18.7 ±0.2, 19.4±0.2, 20.6±0.2, 21.0±0.2, 22.1±0.2, 24.2±0.2, 27.0±0.2, 28.1±0.2, 29.5±0.2, 30.2±0.2, 30.9±0.2, 33.1±0.2, 35.2±0.2, 36.8±0.2.

The present invention provides a crystalline polymorphic Form A of Apixaban having a X-ray diffraction spectrum essentially the same as the X-ray powder diffraction spectrum shown in FIG. 1.

X-ray powder diffraction patterns are obtained by irradiation with CuKα radiation, preferably CuKα λ=1.5418 Å, preferably at a temperature of about 22°C.

The present invention provides a crystalline polymorph Form A of apixaban characterized by endothermic melt peak (melting point) at 235°C determined by DSC.

Another embodiment relates to a crystalline Form A of apixaban having a differential scanning calorimetry (DSC) thermogram essentially the same as that shown in FIG. 2.

A crystalline polymorphic Form A of apixaban is provided in substantially pure form. This crystalline form of apixaban in substantially pure form may be employed in pharmaceutical compositions which may optionally include one or more other components selected, for example, from the group consisting of excipients, carriers, diluent and one of other active pharmaceutical ingredients active chemical entities of different molecular structure.

In preferred embodiment, a composition is provided consisting essentially of the crystalline Form A of apixaban. The composition of this embodiment may comprise at least 90 weight % of the crystalline Form A of apixaban, based on the weight of apixaban in the composition.

Preferably, the crystalline form has substantially pure phase homogeneity as indicated by less than 10%, preferably less than 5 %, and more preferably less than 2 % of the total peak area in the experimentally measured PXRD pattern arising from the extra peaks that are absent from the simulated PXRD pattern. Most preferred is a crystalline form having substantially pure phase homogeneity with less than 1% of the total peak area in the experimentally measured PXRD pattern arising from the extra peaks that are absent from the simulated PXRD pattern.

In one preferred embodiment of the invention, the crystalline Form A of apixaban that has a significantly higher solubility compared to polymorphic forms known in the state of the art is provided.

Preferably, the solubility of crystalline Form A of Apixaban in aqueous media at pH 1.2 at 37°C is more than 55 µg/ml. Preferably, the solubility of crystalline Form A of apixaban in aqueous media at pH 4.5 at 37°C is more than 70 µg/ml. Preferably, the solubility of crystalline Form A of apixaban in aqueous media at pH 6.8 at 37°C is more than 50 µg/ml more preferably more than 60 µg/ml, even more preferably more than 70 µg/ml. Even more preferably, the solubility of crystalline Form A of apixaban in aqueous media at 37°C at pH 4.5 more than 70 µg/ml and at pH 6.8 more than 50 µg/ml. Most preferably, the solubility of crystalline Form A of apixaban in aqueous media at 37°C at pH 1.2 is more than 55 µg/ml, at pH 4.5 more than 70 µg/ml and at pH 6.8 more than 70 µg/ml.

For a pharmaceutical use a solubility of the form which active pharmaceutical ingredient forms is a criterion of exceptional importance under certain circumstances depending on the choice of formulation or the choice of manufacturing process. For drugs which are to be taken orally it is, in general, very important that active pharmaceutical ingredients are sufficiently soluble and bioavailable. Apixaban is such a drug, i.e. characterized by a low solubility in aqueous media. Unexpectedly, improved solubility of crystalline polymorph Form A of apixaban contributes to sufficient bioavailability of apixaban containing drugs. At the same time, a high physical stability of crystalline Form A is preserved. Namely, after 8 months samples of crystalline Form A of apixaban stored at room atmosphere conditions the crystalline structure remains unchanged.

The crystalline Form A of apixaban of the disclosure may be used alone or in combination, or formulated with one or more excipients or other active pharmaceutical ingredients to provide formulations suitable for the treatment of the indications identified above.

The crystalline polymorph Form A of apixaban is useful in the treatment of thromboembolic diseases.

XRPD and DSC analytical methods were used for characterization of crystalline polymorph Form A of apixaban according to invention. Equilibrium solubility was determined.

### X-ray Powder Diffraction Measurements

The crystalline polymorph Form A of apixaban was characterized by a x-ray powder diffraction pattern (PXRD) using X'Pert PRO MPD apparatus with X'celerator detector (CuKα = 1.5418 Å) in Bragg-Brentano reflection geometry in the range 2-40 °2θ comprising five or more 2θ values selected from the group consisting of diffraction peaks presented in Table 1.

**Table 1: X-ray powder diffraction pattern (PXRD) of crystalline Form A of apixaban.**

| **No.** | **Pos. [°2θ]** | **d-spacing [A]** | **Height [cts]** | **Rel. Int. [%]** |
|---|---|---|---|---|
| 1 | 3.8637 | 22.86901 | 2782.60 | 14.07 |
| 2 | 4.2709 | 20.68942 | 672.49 | 3.40 |
| 3 | 6.2048 | 14.24469 | 686.65 | 3.47 |
| 4 | 7.7106 | 11.46598 | 8158.29 | 41.24 |
| 5 | 8.5211 | 10.37712 | 5913.89 | 29.90 |
| 6 | 9.4359 | 9.37298 | 3126.03 | 15.80 |
| 7 | 10.8520 | 8.15289 | 16991.34 | 85.90 |
| 8 | 11.2767 | 7.84677 | 7588.95 | 38.36 |
| 9 | 11.5961 | 7.63136 | 10405.90 | 52.61 |
| 10 | 12.7951 | 6.91882 | 4192.58 | 21.19 |
| 11 | 13.8348 | 6.40110 | 2733.49 | 13.82 |
| 12 | 14.5858 | 6.07314 | 6251.68 | 31.60 |
| 13 | 15.5171 | 5.71071 | 10245.37 | 51.79 |
| 14 | 16.5606 | 5.35313 | 5947.94 | 30.07 |
| 15 | 17.2017 | 5.15504 | 7070.55 | 35.74 |
| 16 | 17.8850 | 4.95962 | 18374.00 | 92.89 |
| 17 | 18.7081 | 4.74322 | 19781.04 | 100.00 |
| 18 | 19.4313 | 4.56828 | 9328.61 | 47.16 |
| 19 | 20.5651 | 4.31891 | 15144.60 | 76.56 |
| 20 | 20.9536 | 4.23971 | 10044.24 | 50.78 |
| 21 | 22.0939 | 4.02340 | 10625.52 | 53.72 |
| 22 | 24.1683 | 3.68257 | 5623.34 | 28.43 |
| 23 | 26.9534 | 3.30804 | 4494.05 | 22.72 |
| 24 | 28.0607 | 3.17996 | 3003.17 | 15.18 |
| 25 | 29.4697 | 3.03105 | 5188.61 | 26.23 |
| 26 | 30.1952 | 2.95986 | 3027.00 | 15.30 |
| 27 | 30.9062 | 2.89337 | 2681.27 | 13.55 |
| 28 | 33.0900 | 2.70724 | 1961.10 | 9.91 |
| 29 | 35.2201 | 2.54824 | 1360.00 | 6.88 |
| 30 | 36.8227 | 2.44094 | 1321.40 | 6.68 |

### Differential Scanning Calorimetry (DSC)

The DSC instrument used to test the crystalline polymorph Form A of apixaban was a Mettler Toledo Model DSC 1 apparatus. The instrument was calibrated with high purity indium. The accuracy of the measured sample temperature with this method is within about ± 1°C, and the heat of fusion can be measured within a relative error of about ±5%. The sample was placed into the bottom of an open aluminum DSC pan, and measured against an empty reference pan. About 2-6 mg of sample powder was placed into the bottom of the pan and lightly tapped down to make contact with the pan. The weight of the sample was measured accurately and recorded to a hundredth of a milligram. The instrument was programmed to heat at 10°C/min in the temperature range between 30 and 300°C.

The heat flow, which was normalized by a sample weight, was plotted versus the measured sample temperature. The data were reported in units of watts/gram ("W/g"). The plot was made with the endothermic peaks pointing down. The endothermic melt peak was evaluated for extrapolated onset temperature, peak temperature, and heat of fusion in this analysis.

### Solubility

Equilibrium solubility of crystalline polymorphic Form A of apixaban and references apixaban Form N-1 was performed in various aqueous media of buffers of pH 1.2, 4.5 and 6.8 at 37°C. The solubility was measured using "shake flask" method described in European Pharmacopeia. The shaking was carried out at 400 RMP for 24 hours.

**Table 2: Equilibrium solubility of crystalline polymorphic Form A of apixaban and reference apixaban Form N-1 in various aqueous media of buffers.**

| **pH of media (aqueous buffer)** | **Form N-1 [µg/ml]** | **Form A [µg/ml]** |
|---|---|---|
| 1.2 | 44 | 58 |
| 4.5 | 53 | 74 |
| 6.8 | 30 | 72 |

The comparison of equilibrium solubility of apixaban polymorph forms N-1 and A surprisingly shows that the apixaban crystalline polymorph Form A has a higher solubility than reference polymorph Form N-1 of apixaban (Table 2).

New crystalline polymorph Form A of apixaban is in general prepared by heating of a hydrated crystalline Form H3 of apixaban at elevated temperature and subsequent cooling.

The process of heating is controlled by the time of exposure to a defined temperature, while process of cooling is not controlled anyhow. The atmosphere condition is irrelevant and, thus, not determined for any of the process steps.

Preferably, the process of making Form A of apixaban comprises heating hydrated crystalline Form H3 of apixaban at temperature from about 50°C to about 200°C, preferably from about 100°C to about 160°C, and more preferably at about 120°C in a time range from 10 min to 24 h, preferably from 30 min to 12 h, and more preferably from 1 h to 8 h, and subsequently cooling the substance to a room temperature. Preferred is natural cooling, i.e. at room atmosphere conditions to ambient temperature.

The crystalline Form H3 of apixaban is characterized by a x-ray powder diffraction pattern comprising five or more 2θ values selected from the group consisting of 6.1±0.2, 7.0±0.2, 12.1±0.2, 13.6±0.2, 16.1±0.2, 16.3±0.2, 17.5±0.2, 18.1±0.2, 19.1 ±0.2, 21.1±0.2, 21.5±0.2, 22.6±0.2, 24,2±0.2, 29.9±0.2, 30.3±0.2 and 31.0±0.2.

Preferably, the crystalline Form H3 of apixaban is characterized by a x-ray powder diffraction pattern comprising five or more 2θ values selected from the group consisting of 6.1±0.2, 12.1±0.2, 13.6±0.2, 17.5±0.2, 19.1 ±0.2, 21.5±0.2, 22.6±0.2, 29.9±0.2, 30.3±0.2 and 31.0±0.2.

Apixaban Form H3 may be prepared from ethyl ester of 1-(4-methoxyphenyl)-7-oxo-6-[4-(2-oxopiperidin-1-yl)phenyl]-4,5,6,7-tetrahydro-1H-pyrazolo[3,4-c]pyridine-3-carboxylic acid (Formula II), which is disclosed in US 2006/0069258.

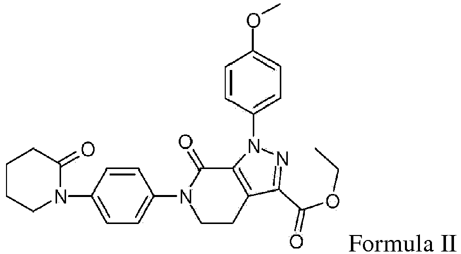

Ethyl 1-(4-methoxyphenyl)-7-oxo-6-[4-(2-oxopiperidin-1-yl)phenyl]-4,5,6,7-tetrahydro-1H-pyrazolo[3,4-c]pyridine-3-carboxylate is mixed with 1,2 propandiol and liquid NH3. A concentration of ester is preferably 5-10 g/mL. Ratio between volume of 1,2 propandiol and NH3 is preferably from 1: 1 to 1:3, more preferably 1:2. The mixture is hated to 60-95°C, preferably to 90°C, and stirred, preferably for 6-18 hours, more preferably 12 hours. Reaction mixture is cooled and a 0.2 fold to 1 fold, preferably 0.3 fold to 0.8 fold, of water with regard to the existing volume of the reaction mixture is added. Precipitate is filtered, washed with water and dried. Obtained product is suspended in ethanol and heated, preferably to temperature from 50°C up to the boiling point of the suspension, more preferably to 70-78°C. After cooling crystalline Form H3 of apixaban is obtained.

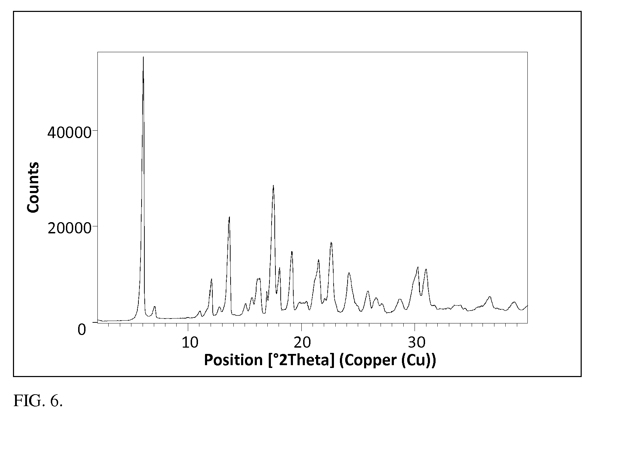

The crystalline Form H3 of apixaban was characterized by a x-ray powder diffraction pattern (PXRD) using X'Pert PRO MPD apparatus with X'celerator detector (CuKα = 1.5418 Å) in Bragg-Brentano reflection geometry in the range 2-40 °2θ comprising five or more 2θ values selected from the group consisting of diffraction peaks presented in Table 3 (FIG. 6.).

**Table 3: X-ray powder diffraction pattern (PXRD) of crystalline Form H3 of apixaban.**

| **No.** | **Pos. [°2Th.]** | **Height [cts]** | **d-spacing [A]** | **Rel. Int. [%]** |
|---|---|---|---|---|
| 1 | 6.0719 | 56761.46 | 14.55618 | 100.00 |
| 2 | 7.0530 | 2992.98 | 12.53349 | 5.27 |
| 3 | 11.0662 | 2082.38 | 7.99557 | 3.67 |
| 4 | 12.0530 | 9032.01 | 7.34305 | 15.91 |
| 5 | 12.7703 | 2972.01 | 6.93216 | 5.24 |
| 6 | 13.6249 | 21238.12 | 6.49921 | 37.42 |
| 7 | 15.0653 | 3660.65 | 5.88092 | 6.45 |
| 8 | 15.6373 | 4967.06 | 5.66705 | 8.75 |
| 9 | 16.1357 | 8502.50 | 5.49313 | 14.98 |
| 10 | 16.3122 | 8503.23 | 5.43408 | 14.98 |
| 11 | 16.9323 | 4480.99 | 5.23212 | 7.89 |
| 12 | 17.5272 | 25670.89 | 5.06004 | 45.23 |
| 13 | 18.0784 | 11252.03 | 4.90700 | 19.82 |
| 14 | 19.1614 | 14201.22 | 4.63203 | 25.02 |
| 15 | 19.7779 | 3860.12 | 4.48901 | 6.80 |
| 16 | 20.4542 | 4085.81 | 4.34207 | 7.20 |
| 17 | 21.1299 | 8227.14 | 4.20473 | 14.49 |
| 18 | 21.5271 | 13027.11 | 4.12804 | 22.95 |
| 19 | 22.0541 | 4104.99 | 4.02724 | 7.23 |
| 20 | 22.6348 | 14568.68 | 3.92846 | 25.67 |
| 21 | 24.1995 | 10203.71 | 3.67789 | 17.98 |
| 22 | 25.8953 | 6451.51 | 3.44075 | 11.37 |
| 23 | 26.6062 | 5017.79 | 3.35041 | 8.84 |
| 24 | 27.1033 | 3364.49 | 3.29008 | 5.93 |
| 25 | 28.6842 | 4704.58 | 3.11225 | 8.29 |
| 26 | 29.8763 | 8142.77 | 2.99072 | 14.35 |
| 27 | 30.3004 | 11533.64 | 2.94982 | 20.32 |
| 28 | 30.9844 | 10881.81 | 2.88625 | 19.17 |
| 29 | 33.8369 | 3309.34 | 2.64918 | 5.83 |
| 30 | 36.6147 | 5038.21 | 2.45432 | 8.88 |
| 31 | 38.7960 | 4074.73 | 2.32120 | 7.18 |

The following non-limiting examples are illustrative of the disclosure.

### EXAMPLES

### Preparation of crystalline polymorph Form H3 of apixaban.

### Example 1

74 g of Ethyl 1-(4-methoxyphenyl)-7-oxo-6-[4-(2-oxopiperidin-1-yl)phenyl]-4,5,6,7-tetrahydro-1H-pyrazolo[3,4-c]pyridine-3-carboxylate (prepared according to US 2006/0069258 A1), 600 mL of 1,2 propandiol and 300 mL of liquid NH₃ were charged in autoclave, heated to 90°C and stirred for 12 hours. 400 mL of water was added to cooled mixture. Precipitate was washed twice with 300 mL of water and dried. Obtained product was suspended in 450 mL of ethanol and suspension was heated. After cooling crystalline Form H3 of apixaban was obtained.

### Preparation of crystalline polymorph Form A of apixaban.

### Example 2.

1 g of apixaban Form H3 was placed in vacuum dryer and dried at temperature of 160°C and 1 bar for at least 4 hours, and cooled down to room temperature. The obtained product was anhydrous Form A of apixaban.

### Example 3.

1g of apixaban Form H3 was placed in aluminum pan and heated at temperature of 120°C for 30 min, and cooled down to room temperature. The obtained product was anhydrous Form A of apixaban.

### Analysis of crystalline Form A of apixaban

### Example 4. X-ray powder diffraction

X-ray powder diffraction (XRPD) data were obtained using X'Pert PRO MPD apparatus with X'celerator detector. The radiation was Cu Kα (λ = 1.5418 Å). Powder samples were flattened in a thin layer on the zero background holder. Variable divergence and antiscatter slits were used to maintain 10 mm of sample length irradiated. Data were collected in the range of 2-40 °2θ with steps of 0.033 °2θ and exposure time of at least 50 seconds per step.

**Table 1: X-ray powder diffraction pattern (PXRD) of crystalline Form A of apixaban.**

| **No.** | **Pos. [°2**θ**]** | **d-spacing [A]** | **Height [cts]** | **Rel. Int. [%]** |
|---|---|---|---|---|
| 1 | 3.8637 | 22.86901 | 2782.60 | 14.07 |
| 2 | 4.2709 | 20.68942 | 672.49 | 3.40 |
| 3 | 6.2048 | 14.24469 | 686.65 | 3.47 |
| 4 | 7.7106 | 11.46598 | 8158.29 | 41.24 |
| 5 | 8.5211 | 10.37712 | 5913.89 | 29.90 |
| 6 | 9.4359 | 9.37298 | 3126.03 | 15.80 |
| 7 | 10.8520 | 8.15289 | 16991.34 | 85.90 |
| 8 | 11.2767 | 7.84677 | 7588.95 | 38.36 |
| 9 | 11.5961 | 7.63136 | 10405.90 | 52.61 |
| 10 | 12.7951 | 6.91882 | 4192.58 | 21.19 |
| 11 | 13.8348 | 6.40110 | 2733.49 | 13.82 |
| 12 | 14.5858 | 6.07314 | 6251.68 | 31.60 |
| 13 | 15.5171 | 5.71071 | 10245.37 | 51.79 |
| 14 | 16.5606 | 5.35313 | 5947.94 | 30.07 |
| 15 | 17.2017 | 5.15504 | 7070.55 | 35.74 |
| 16 | 17.8850 | 4.95962 | 18374.00 | 92.89 |
| 17 | 18.7081 | 4.74322 | 19781.04 | 100.00 |
| 18 | 19.4313 | 4.56828 | 9328.61 | 47.16 |
| 19 | 20.5651 | 4.31891 | 15144.60 | 76.56 |
| 20 | 20.9536 | 4.23971 | 10044.24 | 50.78 |
| 21 | 22.0939 | 4.02340 | 10625.52 | 53.72 |
| 22 | 24.1683 | 3.68257 | 5623.34 | 28.43 |
| 23 | 26.9534 | 3.30804 | 4494.05 | 22.72 |
| 24 | 28.0607 | 3.17996 | 3003.17 | 15.18 |
| 25 | 29.4697 | 3.03105 | 5188.61 | 26.23 |
| 26 | 30.1952 | 2.95986 | 3027.00 | 15.30 |
| 27 | 30.9062 | 2.89337 | 2681.27 | 13.55 |
| 28 | 33.0900 | 2.70724 | 1961.10 | 9.91 |
| 29 | 35.2201 | 2.54824 | 1360.00 | 6.88 |
| 30 | 36.8227 | 2.44094 | 1321.40 | 6.68 |

### Example 5. Differential Scanning Calorimetry

Differential scanning calorimetry was conducted using a Mettler Toledo Model DSC 1 apparatus. For each analysis, the DSC cell/sample chamber was purged with 100 ml/min of ultra-high purity nitrogen gas. The instrument was calibrated with high purity indium. 3.658 mg of the sample powder was placed into the bottom of the open aluminium pan, lightly tapped down to make contact with the pan, and measured against an empty reference pan. The heating rate was 10°C/min in the temperature range between 30 and 300°C. The heat flow, which was normalized by sample weight, was plotted versus the measured sample temperature. The data were reported in units of watts/gram ("W/g"). The plot was made with the endothermic peaks pointing up (FIG. 2.). The endothermic melt peak (melting point) was evaluated for extrapolated onset temperature, which is 235°C.

### Example 6. Solubility determination

Equilibrium solubility of crystalline polymorphic Form A of apixaban and references apixaban Form N-1 was performed in various aqueous media of buffers of pH 1.2, 4.5 and 6.8 at 37°C. The solubility was measured using "shake flask" method described in European Pharmacopeia. The shaking was carried out at 400 rmp for 24 hours.

**Table 2: Equilibrium solubility of crystalline polymorphic Form A of apixaban and reference apixaban Form N-1 in various aqueous media of buffers.**

| **pH of media (aqueous buffer)** | **Form N-1 [µg/ml]** | **Form A [µg/ml]** |
|---|---|---|
| 1.2 | 44 | 58 |
| 4.5 | 53 | 74 |
| 6.8 | 30 | 72 |

### Preparation of pharmaceutical composition comprising crystalline Form A of apixaban.

### Example 6. Pharmaceutical composition

**Table 4: immediate release formulation of crystalline Form A of apixaban.**

| | | **per tbl [mg]** | **% tbl** | |
|---|---|---|---|---|
| | **TABLET CORE** | | | |
| | **INTRAGRANURAL** | | | |
| 1 | APIXABAN FORM A | 2.50 | 2.42 | Active ingredient |
| 2 | LACTOSE ANHYDROUS | 52.50 | 50.72 | Filler |
| 3 | MICROCRYSTALLINE CELLULOSE | 40.00 | 38.65 | Filler / binder |
| 4 | CROSCARMELLOSE SODIUM | 3.00 | 2.90 | Disintegrant |
| 5 | SODIUM LAURYL SULPHATE | 1.00 | 0.97 | Wetting agent |
| 6 | MAGNESIUM STEARATE | 1.00 | 0.97 | Lubricant |
| | **mass of core tablet** | **100.00** | **96.62** | |
| **9** | **FILM COAT** (lactose monohydrate, HPMC, titanium dioxide, triacetin, yellow iron oxide) | 3.50 | 3.38 | |
| | **mass of film coated tablet** | **103.5** | **100.00** | |

Form A of apixaban was mixed with sodium lauryl sulphate, anhydrous lactose, microcrystalline cellulose and crosscarmelose sodium, and sieved through appropriate sieve. The blend was then lubricated with the sieved magnesium stearate (blended for short period of time). The lubricated blend was then compressed into tablets and film coated.

## Claims

1. A crystalline Form A of apixaban.

2. A crystalline Form A of apixaban **characterized by** a x-ray powder diffraction pattern comprising five or more 2θ values selected from the group consisting of 3.9±0.2, 7.7±0.2, 8.5±0.2, 10.9±0.2, 11.6±0.2, 15.5±0.2, 17.9±0.2, 18.7 ±0.2, 20.6±0.2, 22.1±0.2 and 29.5±0.2.

3. A crystalline Form A of apixaban **characterized by** a x-ray powder diffraction pattern comprising 2θ values at 10.9±0.2, 17.9±0.2, 18.7 ±0.2, 20.6±0.2 and 22.1±0.2.

4. A crystalline Form A of apixaban according to claim 2 further **characterized by** a x-ray powder diffraction pattern comprising 2θ values at 3.9±0.2, 7.7±0.2, 8.5±0.2, 11.6±0.2, 15.5±0.2 and 29.5±0.2.

5. A crystalline Form A of apixaban having a X-ray diffraction spectrum substantially the same as the X-ray powder diffraction spectrum shown in FIG. 1.

6. A crystalline Form A of apixaban having a differential scanning calorimetry (DSC) thermogram substantially the same as that shown in shown in FIG. 2.

7. A pharmaceutical composition comprising the crystalline Form A of apixaban according to any one of claims 1-6 and a pharmaceutically acceptable carrier or diluent.

8. A crystalline Form A of apixaban according to any one of claims 1-6 for use in treating a thromboembolic disease in a mammal.

9. A process of making a crystalline Form A of apixaban comprising:
a) heat apixaban Form H3 at elevated temperature; and
b) cooling the substance to a room temperature.

10. The process according to claim 9 comprising:
a) heat apixaban Form H3 at temperature from about 50 °C to about 200°C, preferably from about 100 °C to about 160°C, and more preferably at about 120°C in a time range from 10 min to 24h, preferably from 30 min to 12 h, and more preferably from 1 h to 8 h; and
b) cooling the substance to a room temperature.
